# FASCICULE DE BREVET EUROPEEN

(11) **EP 0 552 578 B1**
(45) Date de publication et mention de la délivrance du brevet: **09.07.1997**
(21) Numéro de dépôt: 92403594.2
(22) Date de dépôt: 31.12.1992
(51) Int. Cl.: A61F 2/46, A61B 17/16, A61B 17/17

(54) **Extracteur de ciment chirurgical**
Vorrichtung zum Entfernen von chirurgischem Zement
Surgical cement extractor

(30) Priorité: 03.01.1992 FR 9200013
(43) Date de publication de la demande: 28.07.1993
(73) Titulaire: Bergue, Bertrand, F-37170 Chambray les Tours (FR)
(72) Inventeur: Bergue, Bertrand, F-37170 Chambray les Tours (FR)
(74) Mandataire: Moncheny, Michel

(56) Documents cités:
- EP-A- 0 206 777
- DE-A- 2 542 056
- DE-A- 3 018 491
- GB-A- 2 080 113
- US-A- 4 633 862
- US-A- 4 860 735

## Description

La présente invention concerne un extracteur de ciment chirurgical en orthopédie conforme au préambule de la revendication 1.

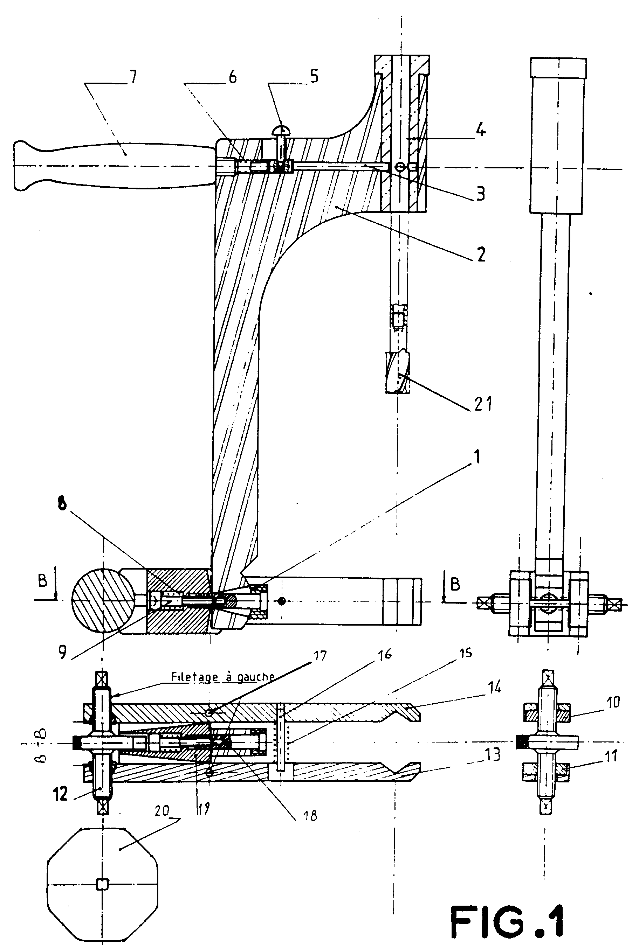

Le document DE-A-30 18 491 décrit un dispositif pour retirer le ciment chirurgical d'un fémur en orthopédie. Ce dispositif possède un demi-cadre (Figure 1) comportant sur une de ses extrémités un orifice qui permet le guidage d'outils dans un volume conique dont la base est la partie supérieure du fémur et dont le sommet est centré à une distance adaptée sur l'axe diaphysaire.

Le retrait du ciment dans le fémur est traditionnellement effectué selon trois modes opératoires :
- A l'aide d'instruments classiques le chirurgien (ciseaux longs, crochets) sculpte le ciment progressive-ment jusqu'à l'obtention de la forme souhaitée. Les problèmes essentiels sont la durée de l'opération et le risque de perforation du fémur.
- A l'aide d'un cadre fixé, parallèlement à la diaphyse, avec deux pinces sur le fémur et permettant de réaliser un perçage dans le fourreau de ciment. L'outil utilisé est une mèche de perçage classique. Les problèmes résultant de cette méthode sont :
   . Le centrage de l'outil est obligatoirement réalisé par rapport aux deux pinces et non par rapport à l'axe diaphysaire fémoral interne.
   . En fin de perçage il se produit un risque de vissage de la mèche dans les derniers millimètres de ciment, ce qui risque de détériorer le fémur.
   . Cet appareil permet de réaliser un alésage cylindrique alors que la diaphyse réalise plutôt un cône ouvert vers le sommet du fémur.
- A l'aide d'une frais pneumatique tournant à très haute vitesse (du type frais de dentiste). Le problème est un guidage visuel de la fraise et donc un risque de traverser le fémur radialement.

Le dispositif selon l'invention est conforme à la partie caractérisante de la revendication 1 et permet de remédier à ces inconvénients. Il comporte en effet selon une première caractéristique un demi cadre présentant un orifice, sur son plus petit côté, par lequel est introduit un ensemble comportant un réducteur, servant à guider l'outil, et l'outil lui même (chaque outil est muni de son guide). Cet ensemble est solidarisé avec le demi cadre par l'intermédiaire d'un bloqueur. Ainsi l'outil est guidé par rapport au demi cadre, celui-ci permet de positionner les outils à tout endroit de la diaphyse et de la métaphyse grâce à deux mobilités du demi cadre par rapport au fémur. D'autre part le demi cadre possède un orifice, sur son grand côté, par lequel il s'articule grâce à une liaison sphérique à doigt sur une pince à serrage symétrique à 10cm sur une perpendiculaire à l'axe diaphysaire. L'ensemble vient se fixer et se positionner sur la diaphyse fémorale à environ 20cm du grand trochanter par l'intermédiaire d'encoches. L'ensemble ainsi positionné possède deux mobilités dans deux plans orthogonaux (plan frontal et sagittal). L'outil est guidé mais la direction de travail ainsi que l'avance sera déterminée par le chirurgien en fonction d'une étude préalable des radiographies et au cours de l'opération entre deux passages d'outils. Ainsi l'extrémité de l'outil est centrée par rapport à l'axe diaphysaire fémoral quand il est complètement enfoncé et de plus en plus libre au fur et à mesure que l'on remonte vers la partie supérieure du fémur.Enfin compte tenu du fait que le canal fémoral possède une légère conicité dans sa partie métaphysaire : on utilisera tout d'abord un outil de défonçage de diamètre 10mm deux tailles trois lèvres ayant un angle au sommet de 120° permettant de percer le foureau de ciment, ensuite on utilise des alésoirs deux tailles trois lèvres de diamètre 12mm, 14mm et 16mm que l'on guidera en fonction de l'anatomie du fémur,dans la partie finale de grande conicité on utilise le diamètre 16mm tout en ayant des mouvements circulaires dans des plans parallèles ,enfin pour retirer les coins de ciment restant on utilise un dernier outil de forme tronc conique à sa partie distale et possèdant des dents triangulaires permettant de râper le ciment restant.La surface striée ainsi obtenue permet une meilleure adhésion du nouveau ciment .

Selon des modes particuliers de réalisation:
_ La liaison sphérique à doigt du demi cadre et de la pince peut être réalisée au niveau de l'axe fémoral afin d'éviter le léger défaut de centrage dû à l'excentration de 10 cm de l'articulation par rapport à l'axe diaphysaire.
_ La liaison sphérique à doigt peut être réalisée par deux liaisons pivots dont l'axe de l'une d'entre elles est situé à 10 cm sur une perpendiculaire à l'axe diaphysaire permettant un mouvement de rotation dans le plan frontal et l'axe de la seconde se situe sur l'axe diaphysaire permettant un mouvement de rotation dans le plan sagital en évitant ainsi le petit défaut de centrage.
_ Le réducteur peut être le demi cadre lui-même.
_ L'arrêt en rotation et en translation du réducteur peut être réalisé par un serrage radial.
_ L'arrêt en rotation et en translation du réducteur peut être réalisé par un système à billes et ressorts.
_ Le fémur étant parfois très dissymétrique on peut avoir des calles rapportées à la place des clips.
_ Un seul des trois outils spécifiques peut être utilisé.

Les dessins annexés illustrent l'invention:
_ la figure 1 représente en vue de face ,de dessus et de droite le dispositif selon l'invention.
_ la figure 14 représesente une perspective du dispositif selon l'invention.

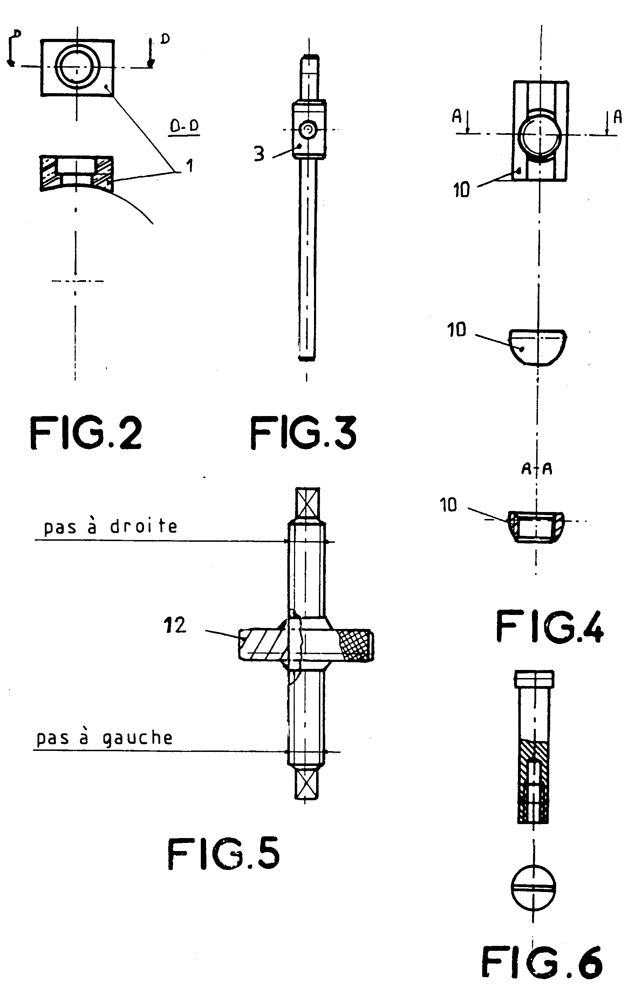

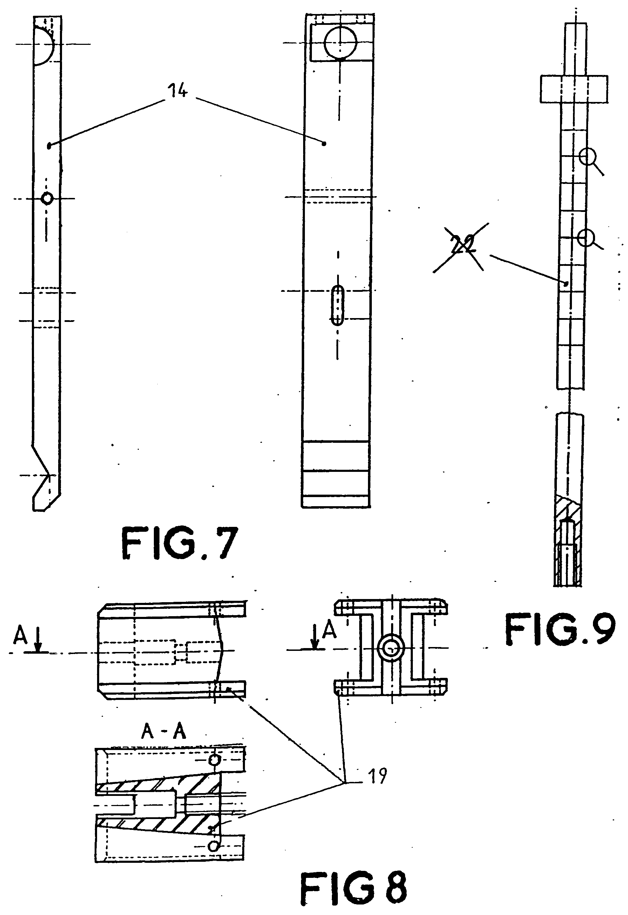

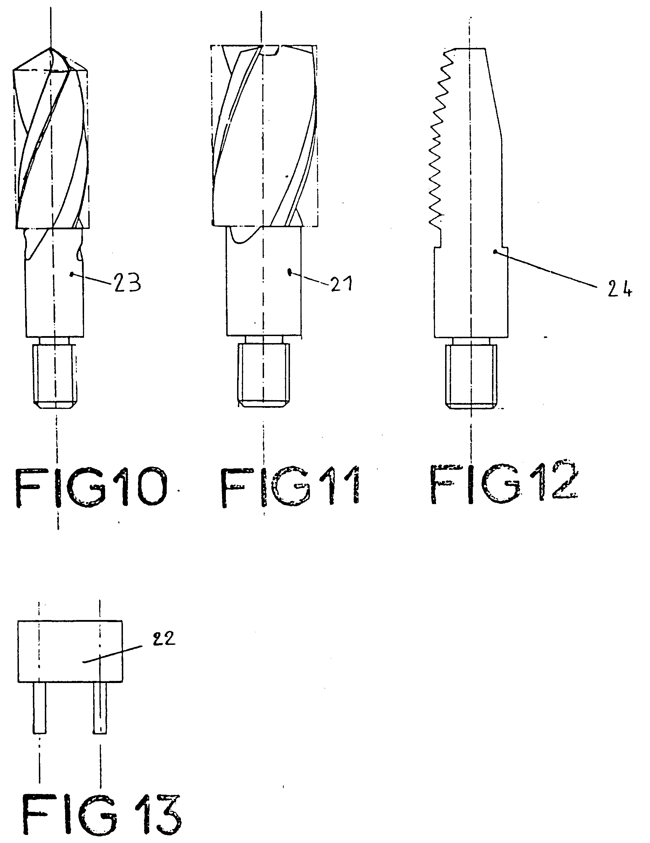

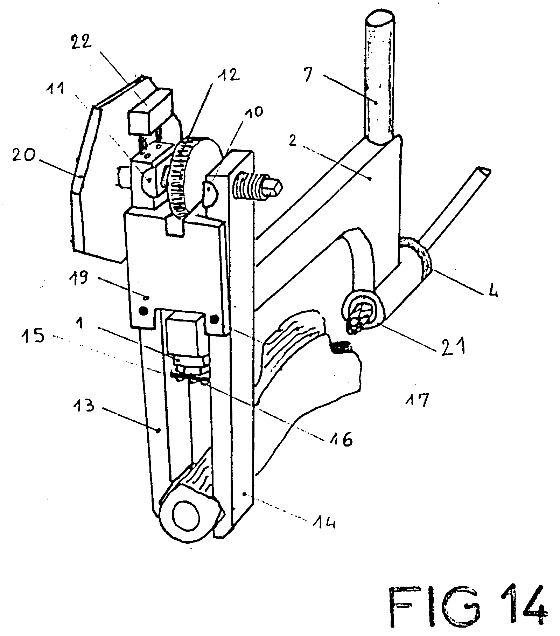

_ les figures 2 à 13 représentent des pièces particulières de l'invention.

En référence à ces dessins,le dispositif comporte un demi cadre (2) muni de deux branches dissymétriques orthogonales.Sur la branche la plus courte le demi cadre (2) possède un alésage qui reçoit le réducteur (4) muni d'une collerette venant en butée sur le demi cadre (2) .Ce réducteur (4) est arrêté en translation et en rotation par un bloqueur (3) venant perpendiculairement à l'alésage s'encliqueter dans l'un des quatres trous réalisés à 90° sur le pourtour du réducteur (4) .Ce bloqueur (3) est maintenu enclenché grâce à un ressort (6) qui exerce un effort sur celui-ci et qui est maintenu sur son autre extrémité dans l'axe du bloqueur (3) par le vissage de la poignée (7) .Le bloqueur (3) peut être retiré par une action du pouce sur le bouton (5) qui est perpendiculaire à l'axe de celui-ci et qui entraine un effort opposé à celui exercé par le ressort (6) et donc un retrait du bloqueur (3) par rapport au réducteur (4).Le réducteur (4) est d'autre part muni d'un outil soit de défonçage ((23) Deux tailles trois lèvres ayant un angle au sommet de 120°) soit à aléser ((21) Deux tailles trois lèvres)) soit de finition ((24) Muni de pointes triangulaires permettant de râper les coins de ciment restant).Sur la plus grande branche du demi cadre se trouve un orifice par lequel est articulé le demi cadre (2) au bloc central (19).Cette articulation étant une articulation sphèrique à doigt , ce qui permet deux mouvements de rotation. Pour l'assemblage on utilise une vis (9) passant dans le bloc (19) sur laquelle il a été préalablement enfilé un ressort (8) et du coté du demi cadre (2) on utilise un tirant (18) sur lequel on a enfilé le patin (1) de frottement ayant une forme arrondie permettant le mouvement du demi cadre (2), le serrage du tirant (18) sur la vis (9) réalise la liaison, le ressort (8) servant de tarrage à l'effort de liaison et laissant ainsi la rotation autour de l'axe de l'assemblage et la rotation par rapport à l'axe de pivotement possible.Sur ce même bloc (19) viennent se fixer en liaison pivot les deux mors de pince (14-13) par les axes (17) sur les faces latérales qui sont obliques et permettent ainsi le mouvement des mors (13-14) .L'écartement des mors (13-14) est réalisé par un ressort (15) guidé sur une goupille (16) fixé sur le mors droit (14) et libre dans un trou oblong sur le mors gauche (13) ,cet ensemble se situant sous l'axe de rotation des mors (13-14) .Le serrage des mors (13-14) est réalisé dans la partie supérieure par une molette (12) munie d'une partie centrale moletée , coté mors droit (14) un filetage à gauche et coté mors gauche (13) un filetage à droite. Cette molette (12) se visse sur deux écrous demi-cylindriques (10-11) pouvant pivoter par rapport aux mors (13-14) lors du serrage et restent ainsi coaxial à la vis de la molette (12). De plus la partie centrale de la molette (12) est guidée dans une rainure sur la partie supérieure du bloc central (19) on obtient un serrage symétrique parfaitement controlé.Enfin sur les extrémités de la molette (12), étant réalisés des carrés , une clé (20) permet d'achever le serrage de la molette (12) en utilisant le principe du bras de levier qui permet d'obtenir un effort de serrage important alors que l'effort de l'utilisateur est moins important. Dans certains cas le fémur étant dissymétrique il est nécessaire d'utiliser un clip (22) guidé sur le mors permettant de caler l'écrou droit (10) ou l'écrou gauche (11) par rapport au mors droit ou au mors gauche (13-14) pour centrer l'outil par rapport à l'axe diaphysaire (le clips se metant sur le mors opposé à la ligne âpre).

Le dispositif selon l'invention est particuliérement destiné au retrait du ciment en orthopédie.

## Revendications

1. Dispositif pour retirer le ciment chirurgical d'un fémur en orthopédie, le dispositif possédant un demi-cadre (2) avec deux extrémités, le demi-cadre (2) possédant sur une des extrémités un orifice avec un axe, cet orifice permettant le guidage d'outils (21, 23, 24) le long de l'axe de l'orifice dans un volume conique dont la base est la partie supérieure du fémur et dont le sommet est centré à une distance adaptée sur l'axe diaphysaire, caractérisé en ce que le demi-cadre (2) est lié et articulé à son autre extrémité à une pince à serrage symétrique venant se fixer sur le fémur, cette articulation étant une articulation sphérique à doigt qui conserve au demi-cadre (2), par rapport à la pince, deux mobilités dans deux plans orthogonaux, le plan frontal et le plan sagittal, la pince étant formée d'un bloc central (19) recevant deux mors (13, 14) en pivot par rapport à celui-ci par l'intermédiaire d'axes (17) dont le serrage est effectué par un système molette (12) écrous (10, 11).

2. Dispositif selon la revendication 1, caractérisé en ce que la liaison sphérique à doigt entre le corps de pince (19) et le demi-cadre (2) est réalisée à 10cm sur une perpendiculaire à l'axe de l'orifice.

3. Dispositif selon la revendication 1, caractérisé en ce que la liaison sphérique à doigt entre le corps de pince (19) et le demi-cadre (2) est réalisée par une articulation à deux mobilités, dans le plan sagittal et le plan frontal, centrée parallèle à l'axe de l'orifice.

4. Dispositif selon la revendication 1, caractérisé en ce que la liaison sphérique à doigt est décomposée en deux liaisons pivots dont l'axe de l'une est à 10cm sur une perpendiculaire à l'axe de l'orifice permettant la mobilité dans le plan frontal et dont l'autre a un axe parallèle à l'axe de l'orifice, permettant la mobilité dans le plan sagittal.

5. Dispositif selon l'une quelconque des revendications précédentes, caractérisé en ce que le guidage des outils (21, 23, 24) est réalisé par un réducteur en bronze (4).

6. Dispositif selon la revendication 5, caractérisé en ce que l'arrêt en rotation et en translation du réducteur est réalisé par un bloqueur (3, 5) ou par un serrage radial ou par un système automatique à billes et ressorts.

7. Dispositif selon l'une quelconque des revendications précédentes, caractérisé en ce qu'il comporte un outil spécifique (23) de défonçage ayant un angle au sommet de 120°, de diamètre 10mm.

8. Dispositif selon la revendication 7, caractérisé en ce qu'il comporte un outil spécifique d'alésage (21), de diamètre 12mm, 14mm ou 16mm.

9. Dispositif selon la revendication 8, caractérisé en ce qu'il comporte un outil spécifique (24) de finition à extrémité distale tronconique muni de petites dents triangulaires.

10. Dispositif selon l'une quelconque des revendications 6 à 8, caractérisé en ce qu'il comporte un seul desdits outils spécifiques (21, 23, 24).

11. Dispositif selon l'une quelconque des revendications précédentes, caractérisé en ce que des clips (22) sont positionnés sur les bras de la pince (13-14) afin de corriger un défaut de coaxialité entre l'axe diaphysaire interne et l'axe de la pince.

12. Dispositif selon l'une quelconque des revendications 1 à 9, caractérisé en ce que des cales sont positionnées sur les mors (13, 14) de la pince afin de corriger un défaut de coaxialité entre l'axe diaphysaire interne et l'axe de la pince.

## Claims

1. Device for removing the surgical cement from a femur in orthopaedics, the device having a half frame (2) with two ends, the half frame (2) having, on one of the ends, an orifice with an axis, this orifice allowing instruments (21, 23, 24) to be guided along the axis of the orifice in a conical volume whose base is the upper part of the femur and whose vertex is centred at a suitable distance on the diaphyseal axis, characterised in that the half frame (2), at its other end, is joined to and hinged on a symmetrical gripping clamp which fixes on the femur, this hinge being a ball and socket hinge with a pin which retains for the half frame (2), with respect to the clamp, two mobilities in two orthogonal planes, the frontal plane and the sagittal plane, the clamp being formed by a central block (19) receiving two jaws (13, 14) which pivot with respect to the latter by way of pins (17), the clamping of which is effected by a system of knurled wheel (12) and nuts (10, 11).

2. Device according to Claim 1, characterised in that the ball and socket joint with a pin between the clamp body (19) and the half frame (2) is formed at 10 cm on a perpendicular to the axis of the orifice.

3. Device according to Claim 1, characterised in that the ball and socket joint with a pin between the clamp body (19) and the half frame (2) is formed by a hinge with two mobilities, in the sagittal plane and the frontal plane, centred parallel to the axis of the orifice.

4. Device according to Claim 1, characterised in that the ball and socket joint with a pin is divided up into two pivoting joints, the axis of one of them being at 10 cm on a perpendicular to the axis of the orifice, permitting the mobility in the frontal plane, and the other of them having an axis parallel to the axis of the orifice, permitting the mobility in the sagittal plane.

5. Device according to any one of the proceding claims, characterized in that the instruments (21, 23, 24) are guided by a bronze reducer (4).

6. Device according to Claim 5, characterized in that the reducer is stopped in rotation and translation by a blocking mechanism (3, 5) or by radial clamping or by an automatic ball and spring system.

7. Device according to any one of the preceding claims, characterized in that it includes a specific breaking instrument (23) having a vortex angle of 120°, and a diameter of 10 mm.

8. Device according to Claim 7, characterized in that it includes a specific boring instrument (21), with a diameter of 12 mm, 14 mm or 16 mm.

9. Device according to Claim 8, characterized in that it includes a specific finishing instrument (24) with a frustoconical distal and equipped with small triangular teeth.

10. Device according to any one of Claims 6 to 8, characterised in that it includes only one of the said specific instruments (21, 23, 24).

11. Device according to any one of the preceding claims, characterised in that clips (22) are positioned on the arms of the clamp (13-14) in order to correct an error in coaxiality between the internal diaphyseal axis and the axis of the clamp.

12. Device according to any one of Claims 1 to 9, characterized in that wedges are positioned on the jaws (13, 14) of the clamp in order to correct an error in coaxiality between the internal diaphyseal axis and the axis of the clamp.

## Patentansprüche

1. Vorrichtung zum Entfernen von chirurgischem Zement aus einem Oberschenkelknochen in der Orthopädie, welche einen Halbrahmen (2) mit zwei Endabschnitten aufweist und dieser Halbrahmen (2) an einem seiner Endabschnitte, eine Öffnung mit einer Achse aufweist, welche die Führung von Werkzeugen (21, 23, 24) entlang der Achse der Öffnung in einem konischen Volumen ermöglicht, dessen Basis der obere Teil des Oberschenkelknochens ist und dessen Spitze in einem geeigneten Abstand auf der Achse der Diaphyse zentriert ist,
**dadurch gekennzeichnet**, **daß**
der Halbrahmen (2) mit seinem anderen Endabschnitt an gelenkig mit einer symmetrischen Spannzange verbunden ist, welche an dem Oberschenkelknochen befestigt wird, und dadurch, daß dieses Gelenk ein kugelförmiges Gelenk mit einem Stellfinger ist, welcher dem Halbrahmen (2) gegenüber der Spannzange zwei Bewegungsmöglichkeiten in zwei orthogonalen Ebenen verleiht, das heißt, in der frontalen Ebene und der sagittalen Ebene, und dadurch, daß die Spannzange aus einem zentralen Block (19) mit zwei beweglichen Spannbacken (13, 14) besteht, welche gegenüber dem zentralen Block (19) mit Hilfe von Achsen (17) verschwenkt werden können, die mit Hilfe eines Systems aus Rändelrädern (12) und Muttern (10, 11) festgezogen werden können.

2. Vorrichtung nach Anspruch 1,
**dadurch gekennzeichnet**, **daß**
das mit einem Stellfinger versehene kugelförmige Gelenk zwischen dem Körper der Spannzange (19) und dem Halbrahmen (2) in einem Abstand von 10 cm auf einer Senkrechten zu der Achse der Öffnung angeordnet wird.

3. Vorrichtung nach Anspruch 1,
**dadurch gekennzeichnet**, **daß**
das mit einem Stellfinger versehene kugelförmige Gelenk zwischen dem Körper der Spannzange (19) und dem Halbrahmen mit Hilfe einer Anlenkung mit zwei Bewegungsmöglichkeiten in der sagittalen und der frontalen Ebene hergestellt wird und daß diese Anlenkung parallel zur Achse der Öffnung zentriert ist.

4. Vorrichtung nach Anspruch 1,
**dadurch gekennzeichnet**, **daß**
das mit einem Stellfinger versehene kugelförmige Gelenk in zwei Schwenkverbindungen unterteilt ist und die Achse der einen Verbindung in einem Abstand von 10 cm auf einer Senkrechten zur Achse der Öffnung angeordnet ist, um dadurch die Beweglichkeit in der frontalen Ebene herzustellen, und daß die andere Verbindung eine parallel zur Achse der Öffnung verlaufende Achse hat, um die Beweglichkeit in der sagittalen Ebene herzustellen.

5. Vorrichtung nach einem der vorausgegangen Ansprüche,
**dadurch gekennzeichnet**, **daß**
die Führung der Werkzeuge (21, 23, 24) mit Hilfe eines Reduzierstückes aus Bronze (4) durchgeführt wird.

6. Vorrichtung nach Anspruch 5,
**dadurch gekennzeichnet**, **daß**
die Arretierung gegen Rotation und Translation des Reduzierstückes mit Hilfe einer Sperre (3, 5), oder durch radiale Verspannung oder mit Hilfe eines automatischen Systems mit Kugeln und Federn sichergestellt wird.

7. Vorrichtung nach einem der vorausgegangenen Ansprüche,
**dadurch gekennzeichnet**, **daß**
sie ein spezielles Fräswerkzeug (23) mit einem Winkel an der Spitze von 120° und einem Durchmesser von 10 mm enthält.

8. Vorrichtung nach Anspruch 7,
**dadurch gekennzeichnet**, **daß**
sie ein spezielles Bohrwerkzeug (21) mit einem Durchmesser von 12 mm, 14 mm oder 16 mm aufweist.

9. Vorrichtung nach Anspruch 8,
**dadurch gekennzeichnet**, **daß**
sie ein spezielles Feinschlichtwerkzeug (24) mit einem distalen stumpfkegeligen Ende enthält, welches mit kleinen dreieckigen Verzahnungen ausgestattet ist.

10. Vorrichtung nach einem der Ansprüche 6 bis 8,
**dadurch gekennzeichnet**, **daß**
sie nur eines der speziellen Werkzeuge (21, 23, 24) aufweist.

11. Vorrichtung nach einem der vorausgegangenen Ansprüche,
**dadurch gekennzeichnet**, **daß**
an den Griffen der Spannzange (13, 14) Krokodilklemmen (22) vorgesehen sind, um eine fehlerhafte Koaxialität zwischen der inneren Achse der Diaphyse und der Achse der Spannzange korrigieren zu können.

12. Vorrichtung nach einem der Ansprüche 1 bis 9,
**dadurch gekennzeichnet**, **daß**
an den Spannbacken (13, 14) der Spannzange Unterlegkeile vorgesehen sind, um eine fehlerhafte Koaxialität zwischen der inneren Achse der Diaphyse und der Achse der Spannzange korrigieren zu können.
